# EUROPEAN PATENT APPLICATION

(11) **EP 4 170 014 A1**
(43) Date of publication of application: **26.04.2023**
(21) Application number: 21203935.8
(22) Date of filing: 21.10.2021
(51) Int. Cl.: C12M 3/06, C12M 1/12, C12M 1/00, C12M 1/34, C12M 1/36

(54) **MICROFLUIDIC SYSTEM FOR ROBUST LONG-TERM ELECTRICAL MEASUREMENT AND/OR STIMULATION OF CELL CULTURES**

(71) Applicant: Alpvision SA, 1800 Vevey (CH); FinalSpark Sarl, 1617 Remaufens (CH)
(72) Inventor: COMBY, Jean-Marc, 1617 Remaufens (CH); MOR, Flavio, 1800 Vevey (CH); KUTTER, Martin, 1800 Vevey (CH); JORDAN, Frédéric, 1800 Vevey (CH)
(74) Representative: Wenger, Joel-Théophile

(57) **Abstract**

A microfluidics system to maintain alive and control cell cultures (100) such as neuronal cell cultures over an air-liquid interface (120) is arranged to prevent the formation of air bubbles and liquid overflows in fully automated 24/7 operation over the mid-long term. A pumping device is adapted to push a liquid through an inlet of the microfluidics channel (130), then through an area of the microfluidics channel that is under the air-liquid interface, then through an outlet of the microfluidics channel, assisted with geometrical arrangements of the microfluidics channel outlet and optionally inlet placements relative to the porous membranes of the air-liquid interface. The pumping device may be programmed with different flow rates, flow directions, and flow durations according to cell culture features and events detected with a computer vision system and/or an electrophysiological signal processing system to automatically adapt the parameters of the pumping device to the current state of the cell culture over the air-liquid interface.

## Description

### TECHNICAL FIELD

The present disclosure generally relates to the field of cell culture devices, more particularly fluid flow assembly arrangements for cell culture, control and monitoring of cell activity.

### BACKGROUND

As an alternative to conventional software and hardware information technology, wetware solutions based on biological components such as cultured cells instead of transistors are now emerging. Rather than replicating high-level cognitive processes with AI using silicon based digital computation, an emerging alternate approach consists of using biological neural networks. Recent progress in biotechnology now facilitates the culture and assembly of biological neural networks out of embryonic stem cells such as rat embryonic stem cells, as well as from differentiated human Induced Pluripotent Stem cells (IPSc). The cultured Biological Neuronal Network (BNN) may then be stimulated and their activity may be recorded using Multi-Electrode Arrays (MEAs). So far, BNNs recorded with MEAs are used in pharmacology studies, pharmaceutical testing and toxicological studies and the like as well as in studies for better understanding common brain diseases such as Alzheimer and Parkinson disease. Other industrial applications have also been proposed in the past few years. An application of BNN to the aircraft control was for instance proposed by DeMarse et al., 2005, Proceedings IEEE International Joint Conference on Neural Networks. US patent 7947626 by the US Navy discloses a passaged progenitor cell derived neural network MEA which may be used to detect and/or quantify various biological or chemical toxins. Along the same track, Koniku (www.koniku.com), the first company to develop "wetchips" as computing chips based on cultured neural cells, has been commercializing highly specialized products since 2017 for the detection of odorant compounds in security, military and agriculture/food markets. As described in their patent application WO 2018/O81657, neuron cells may be modified by various biotechnology processes such as gene editing, methylation editing, and others, to express a unique odorant receptor profile with cell-surface receptors, as known from biology state of the art. Neuron cells may be interfaced with a computer by means of state-of-the-art neurophysiology interfaces, such as MEA (Multi Electrode Arrays) electrodes. One major limitation of such an approach is that it is limited to using adherent, 2D, dissociated neuronal cultures grown on MEAs. Such cultures are arranged in an array of microwells or chambers interconnected by microchannels, using hydrogel and biocompatible polymer arrangements, as described in US2017/015964 and US2021/261898.

US patent application US20140279772 by Baker Hughes discloses the use of a cultured biological neural network in an apparatus for processing signals downhole, conveyed in a container into an earth formation through a borehole. The biological neural network is placed at a container and interfaced with MEA electrodes to receive input signals from sensors and to output measurements out of the neural network. The proposed BNN system also comprises environmental control components, such as an atmosphere recirculator, a carbon dioxide scrubber and an oxygen supply. While this disclosure mentions advantages of BNN over conventional computing systems in a challenging environment in terms of parallel computing power, robustness to vibration and electrical noise, and self-healing capability, it is highly specialized to a specific application, where the preprocessing learning can be applied as an off-line preparation process.

These prior art methods and systems cannot be easily adapted to the long-term 24/7 culture, recording and stimulation of 3D neurospheres or of cerebral organoids. Giandomenico et al., 2019, Nat. Neurosci. 22(4) 669-679 observe that air-liquid interface cerebral organoids (ALI-CO) facilitate the long-term study of *in vitro* cerebral organoids without the need to design a robust automated vascularization system. They demonstrate that the air-liquid interface systems facilitate the continuous nutrient supply that is necessary for the long-term survival of the cerebral organoids. In particular, they report they have tested survival of the ALI-CO for up to one year.

US6130056 describe an ALI device suitable for electrophysiology monitoring of 3D cell cultures that comprise a porous membrane to feed the cells (liquid interface) while providing sufficient oxygenation and CO2 diffusion (air interface). The culture medium passes through the membrane by capillarity and covers the 3D tissue with a film of nutrient liquid. The nutrient medium can be perfused by means of a microfluidics system through a tube under the control of an automatic syringe-driver, at a flow rate usually between 0.1 and 2 µl/min.

US6689594 further describes an improvement of the latter system to facilitate the continuous electrical recording and/or stimulation of the 3D culture with electrodes made directly on a transparent porous membrane on top of the nutrient microfluidics chamber. The membrane may be made of polyethylene terephthalate (PET) or of polycarbonate. The electrodes are arranged below the cell culture, which brings two advantages: 1) the cell culture can by visually monitored without the need to move any element of the arrangement and 2) the cells are pressed against the electrodes under the weight of the nutrient film over them. In particular, this naturally avoids further mechanical manipulation that could otherwise be needed to properly press the living cells in close contact with the electrophysiology measurement hardware.

In "Mass generation, neuron labeling, and 3D imaging of minibrains", Front. Bioeng. Biotechnol. (2021), Govindan et al. describe a step-by-step methodology to generate human minibrain nurseries, culture them and study them by placing them on top of an air-liquid MEA biochip with porous membranes. Minibrains are a brain spheroid model comprising various types of excitatory and inhibitory neurons as well as glial cells, with size of about 500um to 600um, and a lifetime of at least 15 months in proper culture conditions.

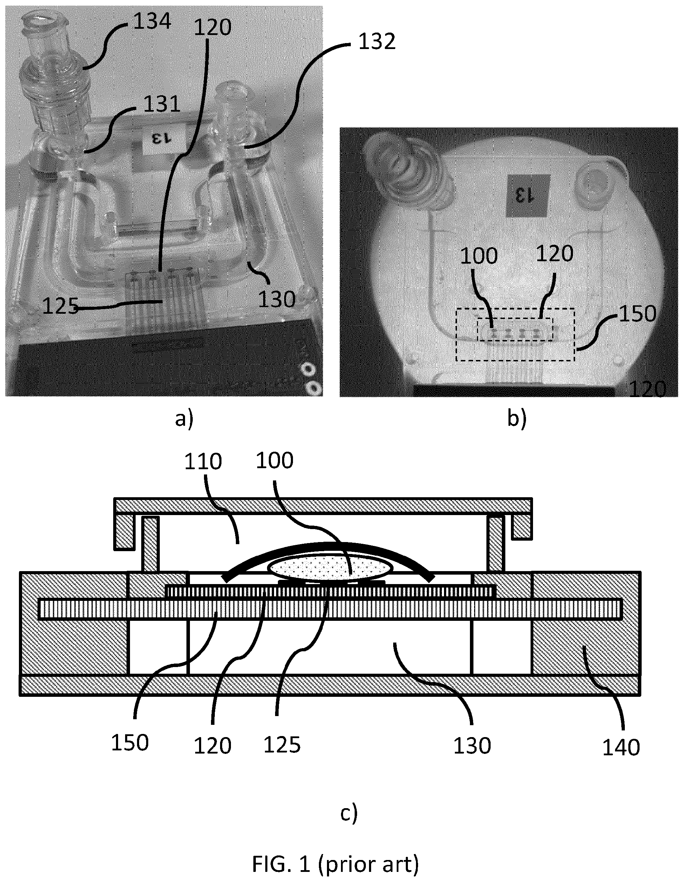

The latter ALI arrangements have also been integrated in a miniaturized "lab-on-chip" device to facilitate the *in vitro* study of functional effects and potential neurotoxicity of chemicals on human brain tissue models *(*Wertenbroek et al., 2021, IEEE Transactions on Biomedical Circuits and Systems 15(4), 743-755*).* FIG. 1 shows a) a general view b) a top view and c) an abstract sectional view of the microfluidics and biochip part of the neuronal cell culture monitoring system of Wertenbroek at al.. Such a system comprises:
- A 3D cell culture 100 of human neural tissue grown from reprogrammed iPS cells, for instance using the protocols of Govindan et al., and placed in a controlled chamber 110 onto a first porous membrane 120 arranged to incorporate the MEA electrodes 125 to form an MEA biochip. The porous membrane 120 is made of polyimide and arranged to be 10% porous by a grid of holes etched through the membrane to facilitate the air-liquid interface. On the MEA biochip of FIG. 1, up to four neurospheres may be cultured and monitor in parallel independently from each other over the porous membrane 120.
- A microfluidics channel 130 to carry the culture medium liquid, built in several layers of PPMA (polymethylmethacrylateplastic) 140, separated from the MEA biochip by a support layer 150 made of a second porous, hydrophobic membrane. The porous, hydrophobic support layer membrane may be made of PTFE (polytetrafluoroethylene) or PET (polyethylene terephthalate) with perforated holes. The fluidic channel carries a flow of liquid from an inlet 131 to an outlet 132 and in particular under the air-liquid interface porous membranes 120, 150 to reach by perfusion the cell culture 100. The inlet 131 and outlet 132 are adapted to facilitate the connection to various ducts and microfluidics systems with standard Luer lock connectors; for instance, a Luer lock-compatible accessory 134 is visible on the inlet 131 in the photo of FIG.1a) while none is present on the outlet 132. For a more robust arrangement, these inlets 131, 132 are preferably built perpendicularly over the fluidic channel PMMA plastic layers horizontal plane, but other arrangements are also possible.
- A fluidics module (not shown) for the automated control of the fluidic perfusion system, comprising a peristaltic pump and a solenoid valve to perfuse the nutrient medium through the fluidic channel 130 onto the 3D cell culture biochip. Alternately, a syringe-driver device may be used instead of a peristaltic pump. For instance, Hamilton syringes may be employed (https://www.hamiltoncompany.com/automated-liquid-handling). The flow direction (usually, aspiration from the cell culture towards the waste collector), the flow rate as well as the time and period of operation are configurable by the fluidics module software to enable different programs operations in a fully automated mode of operation. For instance, the protocol of Govindan et al. recommends the use of intermittent aspiration to replace the liquid nutrient on a regular basis. Note that for neurotoxicity assessment applications specifically, an additional peristaltic pump and solenoid valve may be used for the tested substance distribution system, with their own parametrization.
- A custom-made vision module (not shown) for the optical follow-up of the 3D neural tissues, comprising a camera, a lens and a light source. The vision module can be controlled by a local or a remote computer adapted with different software methods, for instance a computer employing OpenCV open source image processing software, Imaris interactive microscopy imaging software, and/or custom-programmed machine learning tools such as artificial neural networks (ANN) algorithms to facilitate a 24/7 image processing monitoring of the cell culture health by means of pattern recognition, edge detection, color detection, texture detection, cell membrane detection, cell size measurements, cell shape detection, cell counting, etc.
- A neuronal spiking signal recording and analysis module, comprising a hardware board in connection with the MEA biochip (partially shown as the dark component in FIG. 1a)) in combination with a local or a remote computer adapted with different software methods to analyze the cell culture electrophysiology signals. The hardware board of Wertenbroek et al. exemplary setup comprises a microprocessor to manage the signal communications and recordings, and a FPGA to control and process the signals of up to 256 electrodes from the MEA biochip (capable of both recording and stimulation of the 3D neuronal cell culture). The MEA signal processing hardware module can be controlled by a local or a remote computer adapted with different software methods, for instance a computer employing spike analysis algorithms to facilitate the study of the cell culture electrical activity by means of pattern recognition, spike detection, time and/or frequency analysis, etc. The local or remote computer in charge with the electrophysiology signal monitoring may be the same or different than the local or remote computer in charge with the vision module image monitoring.

### PROBLEMS TO BE SOLVED

The exemplary prior art neuronal culture monitoring system from Wertenbroek et al. may thus in theory be adapted for providing the long-term 24/7 continuous operation of a Biological Neuronal Network processing unit for artificial intelligence and cognitive wetware systems, as described for instance in WO2020/049182. However, initial experiments showed it still suffers from a practical limitation when tested over the long-term in a fully automated operation, due to the random development of different disturbances in the microfluidics system. In general, any cell culture integrity on the air-liquid interface is important for cell survival and any disruption in relation with the amount of liquid in contact with the cell culture thereof may be harmful. This includes disruptions of the electrical activity of the cells due to excess variations of the nutrient liquid reaching the cells. In particular, any liquid overflow or underflow, as well as any gaz bubbles around the ALI membranes should therefore be avoided. The origin of the bubbles in fluidics channels is complex and there is currently no standard protocol to prevent their formation (Pereiro et al. , Lab Chip, 2019, 19, 2296-2314*).* In general, the direction of the medium flow may be of critical importance:
- If pumps are used to pull the culture medium (placed downstream, that is beyond the cell culture perfusion along the microfluidics channel circulation direction), the pressure in the medium below the membrane is decreased which may facilitate the formation of bubbles as excess air enters the medium from the air liquid interface above the porous membranes. Ultimately, the cell culture may dry to death due to excess air.
- If pumps are used to push the culture medium (placed upstream before the cell culture perfusion along the microfluidics channel circulation direction), the pressure below the membrane is increased, which may result in excess liquid traversing the porous membranes and going in the chamber where is the BNN. Ultimately, the cell culture may be pushed away from its contact with the MEA biochip electrodes, thus disrupting the stability of the cell culture electrophysiological measurements overtime.

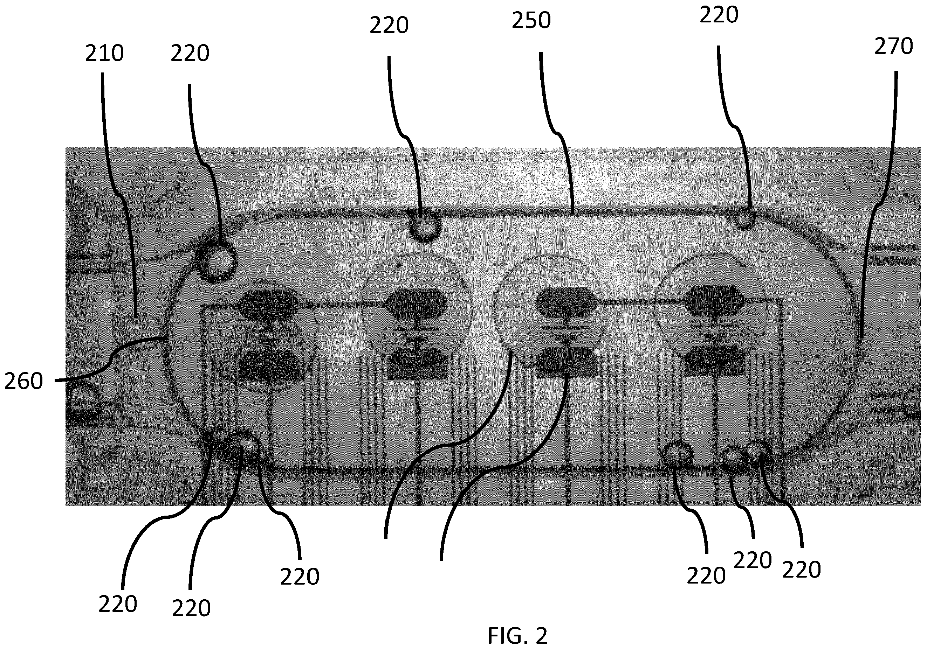

FIG. 2 shows two examples of such bubbles as may be observed by the automated vision module:
- 3D bubbles 220, which are formed in the fluidics channels and flow with the liquid stream from the upstream fluidics channel 260 to the downstream fluidics channel 270, possibly causing disturbances to the ALI system;
- 2D bubbles 210, which appears to be "flatter" bubbles formed between the upper porous polyimide membrane and the lower hydrophobic porous membrane of the air-liquid interface setup, thus preventing the perfusion of the nutrient liquid towards the neuronal cells in the culture laid down above the air-liquid interface membranes.

The inventors have tried multiple experiments with state-of-the-art debubbler solutions, such as the integration of a debubbler device (Elveflow peek bubble remover) upstream in the microfluidics system between the nutrient medium source and the MEA biochip device. While this arrangement filters out some of the bubbles in the nutrient stream before they reach the MEA biochip own air-liquid interface membranes, they observed that not all bubbles are removed and in particular the 2D bubbles which are specific to the use of this air-liquid interface to facilitate the 24/7 MEA biochip signal monitoring. In fact, a large-part of microfluidic debubbler solutions in the field of cellular culture themselves use an air-liquid interface to remove bubbles via differential support of liquid and air through gas-permeable membranes (*"*A low.cost, rapidly integrated debubbler (RID) module for microfluidic cell culture applications", Williams et al., Micromachines 2019, 10, 360). They are thus well suited for conventional cell cultures in which the cells are immersed into a chamber or a microwell full of nutrient liquid. However, they do not efficiently address the specific needs of neuronal cultures that need to lay down on top of the MEA biochip electrodes, and in particular to 24/7 automated neuronal culture systems which employs an air-liquid interface to facilitate the long-term survival of the 3D neurospheres or cerebral organoids over a biochip.

Other solutions in the prior art for microwell arrays rely on coating the microfluidics channel walls and cavities with various components, for instance using a low-cost monosaccharide in Wang et al., Lab Chip, 2012, 12, 3036-3039) or in WO2017180544. Such solutions do not scale well to fit the need for a 24/7 wet operation of an automated cell culture monitoring system.

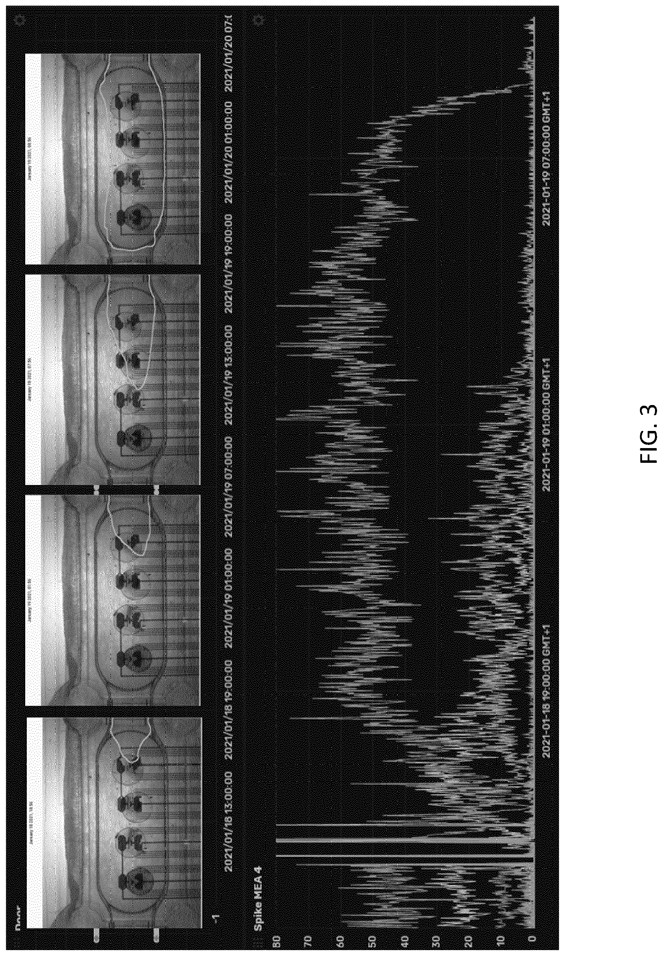

FIG. 3 illustrates the devastating effect of a 2D bubble as visible in the porous membranes of an MEA biochip arrangement enabling the parallel monitoring of 4 different cells cultures under the same microfluidic channel nutrient feed. The custom-made vision module captured images of the MEA biochip cell cultures at different time intervals over a 48 hours period, and automatically recognized the 2D bubble appearing on the right side of the arrangement (recognizable with the automated white line bubble contour detection and marking). From the earliest snapshot on the left to latest one in the right, the increase of the 2D bubble is clearly visible in the images: it finally reaches all the MEA cultures. In parallel, the spike signal monitoring from the MEA4 culture (that is the earliest impacted cell on the right of the setup) is displayed below the images of cell culture monitoring in FIG.3. The spike signals are progressively disrupted and slowed down until the neuron cells are dead and only low-level background electrical residual noise remains visible in the signals. In this experiment, all 4 cultured neurospheres ultimately died out of nutrient in less than 48 hours due to the development of the devastating 2D bubbles in their air-liquid interface. In other unfortunate experiments, the inventors observed the disruption of the signal in less than 15 minutes and the death of the cells in less than 1 hour, due to an out-of-control 2D bubble formed in the double porous membrane.

In contrast to the present application, prior art applications of functional effects testing for toxicity studies and pharmacology purposes do not require 24/7 signal monitoring. As described for instance, in Wertenbroek et al., at the end of a 5-minute exposure period to a substance to be tested, the culture medium is perfused for an additional 4 minutes to wash out the remaining components of the substance (possibly including the bubbles), and the electrical testing only takes place after the wash out is completed. With such setups, it is common practice to automatically pull the nutrient liquid with a peristaltic pump or syringe-driver placed downstream after the microfluidics outlet on the nutrient liquid distribution system. The takes the liquid away from the perfusion MEA biochip area rather than flooding it onto it. If a gas bubble is created around the ALI membranes in the early stages of the test, it is still possible for the automated vision module to detect it and to stop the testing procedure until it is fixed, either by calling for a manual operation, or by adapting, with the overall microfluidics system controller, the washout operation (for instance, by slightly increasing the perfusion flow rate until the bubble is washed out, in a duration short enough not to create an irreversible overflow of the neuronal cell culture).

This detect-and-fix process is however not possible in the 24/7 signal monitoring applications as any bubble may impact the signal processing operations as early as it is formed. The inventors tested multiple arrangements for the microfluidics circulation system, with either a peristaltic pump or a syringe-driver automated system, and multiple methods of operating the nutrient medium circulation in the microfluidics channel without finding an efficient, durable 24/7 solution. Examples of peristaltic pumps include the precise peristaltic pump BT100-2J manufactured by Longer, which can be programmed to deliver flow rates between 0.2µl/min and 380ml/min, the robust industrial peristaltic pump G100-1J also manufactured by Longer, which can be programmed to deliver any flow rate up to 500ml/min, or various peristaltic pump models manufactured by Darwin Microfluidics, which can be programmed to deliver any flow rate between 1.5-2µl/min and 367-380ml/mn. Examples of syringe pumps include the SyringeOne series manufactured by NewEra Instruments, which can be programmed to deliver flow rates from 0.008 nl/hr to 2545µl/min depending on the model, or various syringe pump models manufactured by Darwin Microfluidics, some of which can be programmed to deliver a broad range of flow rates, possibly as low as about 0.5 pl/min.

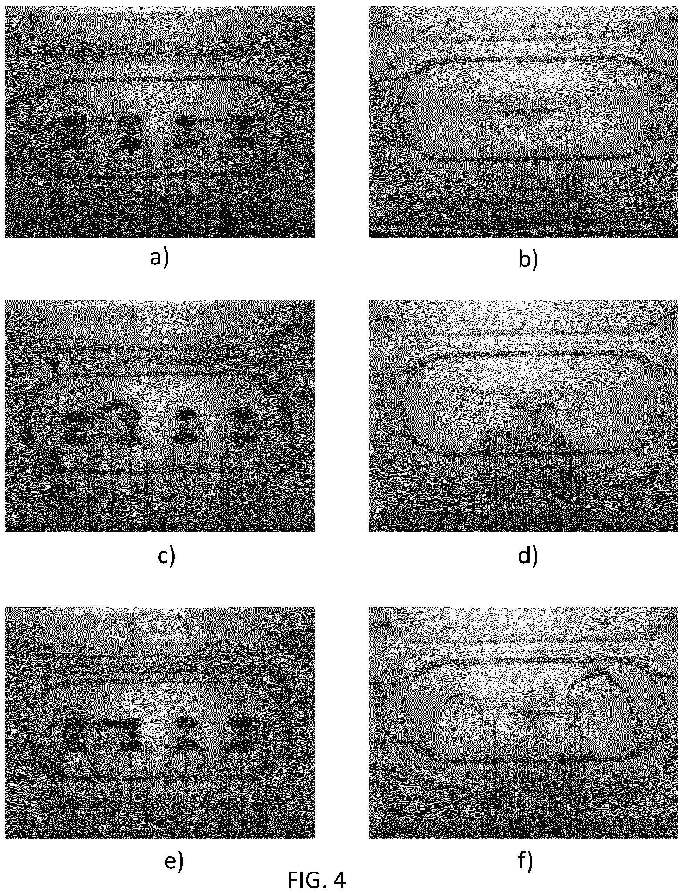

The inventors observed that using a peristaltic pump to perfuse the MEA biochip in pull mode (aspiration) increases the size of both the 2D and the 3D bubbles once they have formed in the system. Pushing the bubbles away from the air-liquid interface by slightly increasing the perfusion flow rate until the bubble is washed away from the device may be efficient in getting rid of the bubbles, but this requires to supply the nutrient liquid too fast towards the biochip. This increases the risk of a liquid overflow over the air-liquid interface. In other words, in order to fix the problem of too much air due to the bubbles in the ALI, a new problem is created with too much liquid due to the overflow. This excess of liquid on the ALI causes another functional disruption in their signals, or even their death. In particular, the inventors observed that any liquid overflow tends to displace the neurospheres out-of-control, thus causing another source of disruption in their continuous MEA signal monitoring. FIG. 4 shows examples of overflow consequences using two different MEA biochip cultures on the left and on the right respectively: a) and b) normal operation, no overflow; c) and d) start of overflow can be visually detected; e) and f), harmful effects of the overflow on the cell cultures are visible as some neurospheres are displaced out of contact from the MEA biochip electrodes.

New solutions are therefore needed for more robust long-term automated neuronal cell cultures with MEA biochips with an air-liquid interface which minimizes the formation of bubbles at the interface.

### BRIEF SUMMARY OF THE INVENTION

It is an object of the present disclosure to provide improved 3D cell culture monitoring devices, system and methods to prevent the signal processing disruption as well as the deterioration or even death of the 3D cultured cells due to gas bubbles and liquid overflows as may be formed in the microfluidics system.

A microfluidic system is described for maintaining of at least one cell culture alive over an air-liquid interface, comprising a microfluidics channel arranged to provide a perfusion of liquid to said cell culture through one or more porous membranes of the air-liquid interface, a pumping device arranged to circulate a liquid through the microfluidics channel, characterized in that the pumping device is adapted to push a liquid successively through an inlet of the microfluidics channel, then through an area of the microfluidics channel that is under the air-liquid interface, then through an outlet of the microfluidics channel.

In a possible embodiment, the outlet of the microfluidics channel are placed at the same height or lower than the porous membranes of the air-liquid interface in the microfluidics system. In a further possible embodiment, the microfluidics system comprises a waste collector to collect the liquid pushed from the outlet of the microfluidics channel, and the waste collector and any microfluidics elements connecting the outlet to the waste collector are placed at the same height or lower than the porous membranes of the air-liquid interface in the microfluidic system. In a further possible embodiment, the microfluidic channel and the air-liquid interface are tilted such that the microfluidics channel outlet is placed at the same height or lower than the porous membranes of the air-liquid interface. In a further possible embodiment, the microfluidic channel and the air-liquid interface are arranged on a rigid planar support in the microfluidic system, and the rigid planar support is tilted such that the microfluidics channel outlet is placed at the same height or lower than the porous membranes of the air-liquid interface. In a still further possible embodiment, the inlet of the microfluidic channel is placed lower than the outlet of the microfluidic channel in the microfluidic system. In a further embodiment, the microfluidic channel is tilted such that the inlet of the microfluidic channel is placed lower than the outlet of the microfluidic channel in the microfluidic system. In a further embodiment, the microfluidic channel is arranged on a rigid planar support in the microfluidic system, and the rigid planar support is tilted such that the inlet of the microfluidic channel is placed lower than the outlet of the microfluidic channel in the microfluidic system.

The pumping device may be a programmable peristaltic pump, syringe-driver, or microgear pump. In a possible embodiment, the pumping device is programmed to push the liquid at a mean flow rate between around 10µl/hr and around 100µl/hr. In a further possible embodiment, the programmable pumping device is programmed to push the liquid at a flow rate between around 10µl/mn in intermittent mode for around 1mn every 10mn.

In a possible embodiment, the microfluidic system further comprises a central processing system adapted to detect, with the computer vision image processing system, an image feature and a central processing system, adapted to program the pumping device flow direction, flow rate and flow duration according to the detected image feature. In a further possible embodiment, the microfluidic system may detect, with a trained machine learning model, an air bubble or a liquid overflow, and the central processing system may program the pumping device flow direction, flow rate and flow duration with a predetermined configuration according to the detected air bubble or liquid overflow. In a possible embodiment, it may program the pumping device flow direction, flow rate and flow duration according to a configuration learnt by the trained machine learning model in response to the detected air bubble or liquid overflow.

In a further possible embodiment, the microfluidic system may also comprise an electrophysiology signal processing system, adapted to detect, with the electrophysiology signal processing system, a signal feature of the cell culture electrophysiological signal; and the central processing system may be adapted to program the pumping device flow direction, flow rate and flow duration according to the detected signal feature.

### SHORT DESCRIPTION OF THE FIGURES

FIG. 1) illustrates an exemplary prior art air-liquid interface (ALI) cell culture device witn an MEA biochip to enable long-term electrophysiology measurements of the cell culture electrical activity (HEPIA SpikeOnChip) a) perspective view of the exemplary ALI MEA biochip device b) top view, by transparency, of the MEA biochip area c) abstract ectional view of the main functional layers of the ALI cell culture, from bottom to top.
FIG. 2) shows examples of disrupting 2D and 3D bubbles in top view images of the MEA biochip and an ALI in operation.
FIG. 3) shows an example of the devastating impact of a developing 2D bubble on top view images of a MEA biochip arrangement in operation and on the monitored spiking signal from one of the neuronal cell culture on the MEA biochip over a period of 48 hours from the apparition of the bubble until the death of the neuronal cell culture.
FIG. 4) shows photographs at different timestamps of a) b) two MEA biochip systems in normal operation and c) d) e) f) the same MEA biochip systems disrupted by a liquid overflow event.
FIG. 5) shows a) a 3D view of a possible support device to optimize the placement of the ALI cell culture device MEA biochip area and fluidics elements such that the fluidics channel outlet is positioned at a lower height than the MEA biochip area.
FIG. 6) shows a 3D view of a possible support device to further optimize the placement of the ALI cell culture device MEA biochip area and fluidics elements along a combination of two slopes: a first slope such that the fluidics channel outlet is positioned at a lower height than the MEA biochip area and along a second slope such that the part of the microfluidics channel below the ALI cell culture is inclined with the input duct at a lower height than the output duct to facilitate the elimination of bubbles from this part of the microfluidics channel towards the microfluidics channel outlet.
FIG. 7) illustrates the main elements of a 24/7 automated cell culture monitoring system..
FIG. 8) illsutrates a 24/7 neuronal cell culture laboratory employing four different arrangements of the proposed system.
FIG.9) shows two photos of the same MEA biochip at two diffferent times, wherein a potientially disrupting 2D bubble has been removed.

### DETAILED DESCRIPTION

A biochip device is understood to be a device arrangement comprising electronics in connection with a chamber, a well or more generally any suitable biocompatible geometry to host a cell culture in close contact with the electronics components such as electrical wires, sensors, or electrodes. An MEA **biochip** is understood to be a device arrangement comprising a micro-electrode array (MEA) in connection with a chamber, a well or more generally any suitable biocompatible geometry to host a cell culture in close contact with the MEA electrodes.

It is understood that a 3D cell culture, in particular a 3D culture of human neural tissue, such as a neurosphere, a cerebral organoid, a cortical organoid, also called a "mini-brain", or any other organic tissue capable of electrophysiological activity such as cardiac tissue, muscular tissue, and other tissues of human or animal origin, may be grown on an **air-liquid interface** (ALI) of a biochip using systems and methods of the prior art.

In a possible embodiment of a MEA biochip arrangement, the cell culture may be monitored on a membrane that incorporates electrodes, called herein an electrode membrane. Electrode membrane may be made of a polyimide membrane or any other suitable material such as biocompatible high performance plastics or polymers. Electrode membrane is arranged to incorporate electrodes such as the MEA electrodes or any other suitable electrodes to capture sample electrophysiological signals from the cell culture. It is preferred that an electrode membrane is porous so that to facilitate the air-liquid interface. Said electrode membrane contains pores ranging from e.g. 0.1 to 10 µm for microfiltration and 0.001 to 0.1µm for ultrafiltration. Said electrode membrane is 5%-15% porous, in particular 10% porous. In a particular embodiment, the electrode membrane is a polyimide membrane that is 5%-15% porous, in particular 10% porous due to a grid of holes etched through the membrane to facilitate the air-liquid interface, but other embodiments are also possible.

It is understood that a 3D cell culture, in particular a 3D culture of human neural tissue, is grown on an electrode membrane that is supported on a second membrane layer, called herein a support layer. Such a support layer is in direct contact with the culture medium. Support membrane may be made of the PET membrane or any other suitable material such as PTFE and similar materials, preferably transparent materials.

It is understood that the homeostasis of a 3D cell culture, in particular a 3D culture of human neural tissue, is maintained over time on the MEA biochip by supplying the necessary gas and **liquid nutrient/cellular waste,** also called **culture medium.** It is understood that a cell culture may be supplied with a culture medium to both the apical and basal compartments of this cell culture, or that the basal surface of the cells is in contact with liquid culture medium, whereas the apical surface is exposed to air.

It is understood that a biochip may be part of a microfluidic system. In particular, the liquid part of an air-liquid interface may be provided by a **fluidics** or **microfluidics channel** arranged to pass under the air-liquid interface in a planar arrangement on the same right plastic support as the biochip device (FIG. 1). A microfluidics channel 130 conveys, by microfluidics circulation of the liquid flow at a predetermined flow rate, the liquid from an upstream liquid dispenser (not represented) to the MEA biochip through an inlet 131. The liquid then reaches the cell culture 100 by perfusion through the air-liquid interface porous membranes 120, 150. The microfluidics channel 130 further conveys, by microfluidics circulation of the liquid flow at the predetermined flow rate, the liquid away from the MEA biochip towards a downstream waste collector (not represented) through an outlet 132. Microfluidics channels, ducts, hoses, pipes, and/or luer lock systems (for instance, 134) are used to interconnect the microfluidics liquid distribution system elements. Microfluidics pumps and optionally valves (not represented) are used to control the flow rate and circulation direction of the liquid through the microfluidics system. Preferably, the microfluidics pumps are programmed so that the liquid generally circulates from the liquid dispenser towards the waste collector, therefore from the inlet 131 to the outlet 132 of the microfluidics channel 130, but they may also be programmed to temporarily invert the flow direction. Optionally, a flow meter may be used to measure the actual flow rate in the microfluidics system.

An air/gas **bubble or bubble** is understood to be globule of gas in a liquid. As used herein, a 2D bubble is a relatively flat bubble formed between two membranes of culture support system and usually has a height of less than 1mm. As used herein, a 3D bubble is formed in the microfluidics channels of a culture setup and usually has a diameter of less than 1mm.

In a 3D culture of human neural tissue a 2D air bubble is thus formed in the double porous membrane, for instance between an electrode membrane and a support layer membrane.

### Optimized "slow-push" pumping

In order to try and get rid of the bubbles, the inventors tried to revert the microfluidics pumping main mode of operation by placing the pump upstream to the microfluidics channel inlet and inverting its flow rate from pull mode (pumping of the liquid from the microfluidics outlet towards the waste collector, resulting in aspiration of the liquid from the nutrient dispenser to the MEA biopchip) to push mode (pumping of the liquid from the liquid dispenser to the microfluidics inlet to supply the MEA biochip).

In one embodiment of the proposed system, a pump device, such as for instance a peristaltic pump, a syringe-driver, or a microgear pump, is placed upstream to the microfluidics channel inlet and operates push mode (pumping of the liquid from the liquid dispenser to the microfluidics inlet to supply the MEA biochip). As will be apparent to those skilled in the art of cell culture over an air-liquid interface, this differs from the conventional setup of placing the pump device downstream from the microfluidics channel outlet to operate in pull mode (pumping of the liquid from the microfluidics outlet towards the waste collector, resulting in aspiration of the liquid from the nutrient dispenser to the MEA biopchip). Preferably, the pumping device is programmed to automatically operate in slow-push mode from a liquid dispenser towards the inlet duct to perfuse the cell culture with nutrient medium at a low flow rate. The medium needs to be pushed towards the cell culture through the microfluidics channel at time intervals regular enough to prevent the formation of bubbles and to feed the cells in accordance with their biological needs. In a possible embodiment, the pumping device operates at a mean flow rate of at most at least around 10µl/hr and at most around 100 µl/hr. In a further possible embodiment, the pumping device may be programmed to operate in intermittent mode. In a possible embodiment, the pump may be programmed to operate at a rate of around 10µl/mn for at least 1 mn every 10 mn. This corresponds to a consumption of 60µl/hr of the nutrient liquid, which is enough to sustain the cell culture perfusion, and it has been experimentally shown to avoid the creation of many 2D bubbles and to facilitate the expelling of large 3D bubbles that may still have formed in the microfluidics circuit. Other embodiments are also possible, for instance for the setup of FIG.1 operating a peristaltic pump at 0.1rpm (corresponding to 10µl/mn) in intermittent mode during for around 1mn to around 5mn during a time period of around every 20mn, or around every 30mn, or around every 45mn, or around every 60mn; or a syringe-driver at a constant flow rate of around 25µl/hr.

### Optimized geometry

With the above "slow-push" mode pumping solution, the bubble problem is generally solved in short-term operations. However, depending on the actual geometry of the end-to-end microfluidics channel, the proposed rate flows may still result into a progressive overflow of the nutrient liquid through the perfusion membrane, as too much liquid is ultimately pushed towards the cell chamber. The inventors observed that the specific geometry of the microfluidics device as shown on FIG. 1, with microfluidics channel inlet and outlet ducts placed perpendicularly above the horizontal plane of the device carrying the MEA biochip at a height of about 1cm above the latter plane, may be improved to decrease the risk of overflow by taking advantage of gravity to compensate for the excessive pressure of the nutrient liquid distribution as may be caused by the pushing mode of operation of the upstream pump.

In a possible embodiment, the MEA biochip device may arranged to operate in a non-horizontal plane (for instance, tilted) such that the outlet of the microfluidics channel is placed at the same height or lower than the air-liquid interface membranes. With this arrangement, any liquid nutrient in excess due to the slow-push operation no longer overflows the cultured cells perfusion but rather more naturally flows below the air-liquid interface towards the microfluidics channel outlet. In other words, gravity helps artificially "pulling" the liquid nutrient away from the MEA biochip, so it is possible fur the pump to push the liquid nutrient towards the MEA biochip with less effort, thus creating less disturbances in the liquid nutrient flow stream.

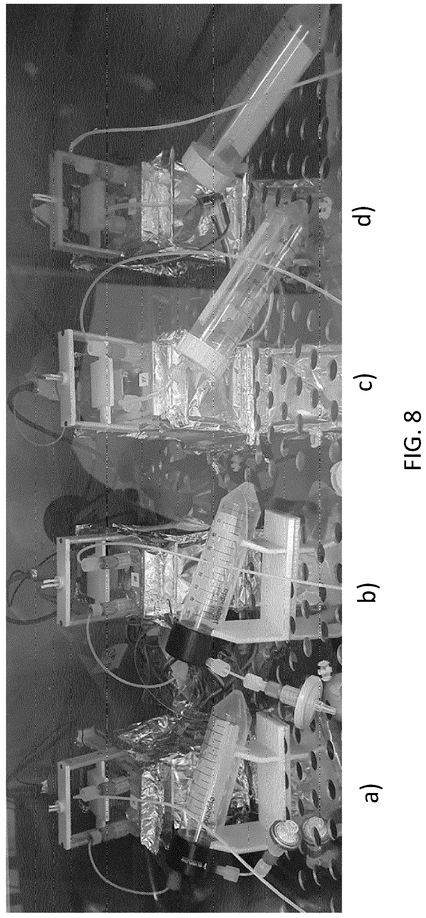

In a further possible embodiment, all elements hosting the expelled nutrient, such as the outlet Luer lock, any hose connecting the outlet to the waste container for the washed-out liquid nutrient, and the waste container itself may be geometrically designed and/or spatially arranged to be placed at the same height or lower than the MEA biochip air-liquid interface membranes in the 24/7 end-to-end system. For instance, a shorter outlet, lock or hose may be employed to remain as small as possible compared to the height of the air-liquid interface membranes; the waste container, for instance a Falcon 50 container, may be itself inclined to shorten its height, as illustrated on FIG. 8. As a consequence, less bubbles, and in particular less 2D bubbles, are formed at the ALI membrane level as the liquid flows more smoothly from the inlet to the outlet duct through the microfluidics channel without the extra pressure of gravity on the nutrient liquid at the altitude of the membrane interface.

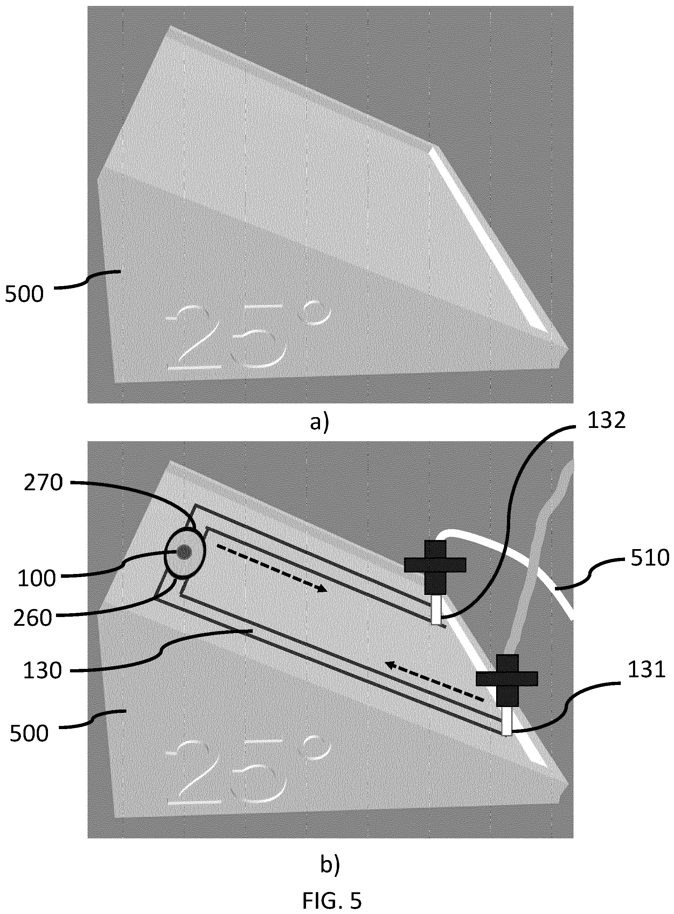

The MEA biochip device may be positioned in a non-horizontal plane with different fixation means. In a possible embodiment, it may be simply placed over a sloped surface support. The sloped surface support may be manufactured at low cost using additive manufacturing techniques, such as 3D printing. FIG. 5a) shows an exemplary 3D view extracted from a sloped surface support model 500 as may be represented in the STL file format suitable for 3D printing. In this embodiment, the sloped surface 500 is arranged with a downward slope along the axis of the microfluidics channel 130 output duct which connects the MEA biochip air-liquid-interface area to the outlet 132. This geometry ensures that the cultured neurospheres still lay down on the MEA biochip with sufficient contact due to automatic gravitational placement, while the microfluidics channel outlet 132 is placed at the same height or lower than the ALI membranes to prevent the formation of bubbles near the cultured cells. As schematically illustrated in FIG. 5b), the output hose 510 may also be arranged with a curvature to minimize its extra height over the Luer lock. More generally, any downstream elements connecting the MEA biochip microfluidics channel outlet to the culture medium waste collector (not shown) may be geometrically arranged to be placed below the MEA biochip ALI area.

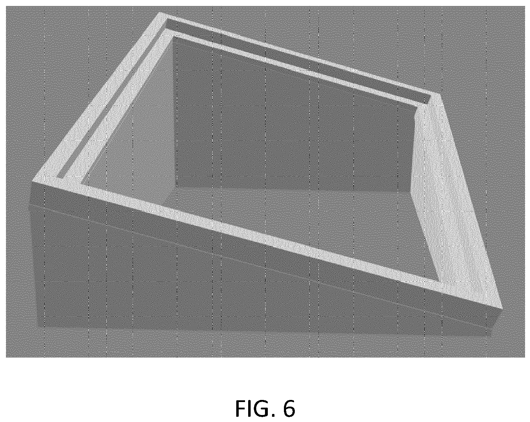

Other arrangements for the slope support are also possible. As shown on FIG.2, 3D bubbles 220 tend to form near the edges of the microfluidics channel duct that is directly placed under the air-liquid interface membranes, in particular when this part of the microfluidics channel is narrower than the input and the output ducts. To compensate for such a geometrical configuration for the MEA biochip device, it may be advantageous to geometrically design a secondary slope along the flow axis of the central part of the microfluidics channel duct below the ALI interface. With reference to the example of FIG. 2, the upstream duct 260 may be preferably placed lower than the downstream duct 270, so that the 3D bubbles are naturally pushed towards the outlet direction. Thus, in a further possible embodiment, the surface supporting the MEA biochip cell culture may be tilted with a combination of a first slope along a first axis along the downstream part of the microfluidics channel from the MEA biochip cell culture to the microfluidics outlet and of a second slope along the axis of the part of the microfluidics duct which lies below the air-liquid interface of the MEA biochip cell culture for the perfusion. This geometrical arrangement may be chosen with a combination of a first slope angle and a second slope angler such that the cultured neurospheres still lay down on the MEA biochip with sufficient contact due to automatic gravitational placement, while facilitating the prevention and the elimination of any 2D or 3D bubbles along the microfluidics channel in particular near and below the critical ALI area of the MEA biochip. More generally, the surface supporting the MEA biochip cell culture may be tilted such that the inlet 131 of the microfluidics channel 130 is placed lower than the outlet 132. FIG. 6 shows accordingly an exemplary 3D view extracted from a double sloped surface support model 500 as may be represented in the STL file format suitable for 3D printing.

### Automated controlled pumping for long-term operation

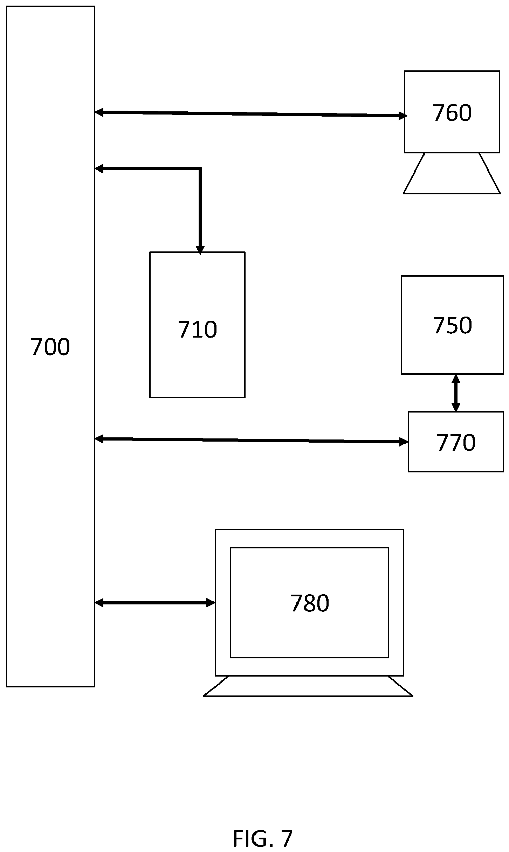

With the above "slow-push" automated pumping solution combined with the optimized geometry of the cell culture monitoring system, the bubble and the overflow problems are generally solved for sustainable 24/7 operations. However, slow drifts in particular with aging pumps have been observed in the mid-long term operations, after several weeks or several months, as well as from time to time unexpected events still causing the formation and uncontrolled development of 2D or 3D bubbles. FIG. 7 shows the main system components of a 24/7 MEA biochip monitoring system comprising:
- A central processing computing system 700, in connection with a user interface 780 for programming the 24/7 operations and reporting the cell culture monitoring results such as reports and alarms to an end user; the central processing system and the user interface may be local to the laboratory or remotely operated through a communication network (not shown).
- An MEA biochip 750, comprising a microfluidics channel 130 to feed a 3D cell culture 100 (not shown).
- A progammable automated pump 710 for circulating a liquid through the microfluidics channel 130 of the MEA biochip 150 to perfuse a 3D cell culture 100 grown on the MEA biochip 150. The pump may be programmed with variable parameters such as pumping flow rate, pumping flow direction, pumping start time, pumping duration from the central processing computing system 700 and may report the state of the pump 710 to the central processing computing system 700.
- A computer vision system 760 to monitor, with a camera, a lens and a lighting system, the cell culture 100 on the MEA biochip 750. The computer vision system receives configuration parameters such as the frequency of image snapshot captures over time from central processing computing system 700 and transmits the captured images in return to the central processing computing system 700.
- An electrophysiological signal recording and stimulation system 770 which records and pre-processes the raw MEA signals from the MEA biochip and transmits them to the central processing computing system 700. The electrophysiological signal recording and stimulation system 770 may also receive the MEA stimulation signal parameters from the central processing computing system 700, when the MEA biochip operates in dual mode (both cell signal recording & cell stimulation).

In a possible embodiment, the proposed neuronal culture automated monitoring system may use a vision system 760, for instance the custom-made vision module of Wertenbroek, or other cell culture vision monitoring systems comprising a camera, a lens and a light source. The vision system 760 may operate in combination with the central processing computing system 700 to monitor in real-time the images from the neuronal cell culture 100 over the MEA biochip 750 in order to detect the development of potentially harmful events from the cell culture images, using computer-implemented methods of image processing.

In a possible embodiment, the central processing computing system 700 may detect, from the images captured with the vision system 760, one or more features such as:
- Bubble features, based on texture, color and/or edge detection algorithms to extract:
   - Presence/absence of bubbles
      ∘ Classification as 2D or 3D bubbles
      ∘ Count of bubbles
   - For each bubble:
      ∘ Position in the MEA biochip area
         ▪ In absolute coordinates in the image plane
         ▪ As a relative distance to the closest neurosphere
         ▪ Detection of presence in certain sensitive areas of the ALI
      ∘ Size
      ∘ Growth rate
      ∘ Motion detection
         ▪ Movement direction
         ▪ Motion speed
      ∘ Deformation
   - Overflow features:
      ∘ Stage of overflow, from minor appearance to major impact, in accordance with the texture, color, and/or edge detection of the perfused neuronal culture
      ∘ Evolution rate

As will be apparent to those skilled in the art of image processing, various algorithms such as those from the OpenCV or Imaris software tool libraries may be used to detect the bubble and/or overflow features from one or more imaging features such as texture, color, edges, mathematical morphology, cell culture confluence, and others.

In a possible embodiment, the proposed neuronal culture automated monitoring system may use an automated electrophysiology monitoring system 770, for instance the custom-made MEA monitoring module of Wertenbroek, or other MEA monitoring systems, in combination with the central processing computing system 700, to monitor in real-time the spiking activity from the neuronal cell culture over the MEA biochip air-liquid interface, and to detect the development of potentially harmful events from the cell culture electrophysiology activity, using computer-implemented methods of electrical signal processing.

In a possible embodiment, the central processing computing system 700 may employ a machine learning system trained to extract features from images, such as a deep learning tool, a convolution neural network tool (CNN), an artificial neural network tool (ANN), or others. In a possible embodiment, a fully automatic convolutional neural network (CNN) based tool such as AlexNet, VGG, GoogleNet, UNet, Vnet, ResNet or others may be used, but other embodiments are also possible. In a possible embodiment, at regular times ― for instance once every 1 5, 10, 20, 30, 60, or 90 minutes -, the central processing computing system 700 may trigger the capture of a snapshot image from the cell culture, with the computer vision module 760, and analyze the image to detect a 2D bubble, a 3D bubble, or an overflow. Three different specialized neural networks may be trained to learn specifically how to recognize a 2D bubble, a 3D bubble, or an overflow respectively, or a single multi-purpose neural network may be trained to jointly label the three events. In a further possible embodiment, the central processing computing system 700 may compare two snapshots at different times to measure the evolution of the 2D bubble, the 3D bubble, and/or the overflow event.

In a possible embodiment, the central processing computing system 700 may detect, from the MEA electrical signals, one or more features such as the mean firing frequency, the signal-to-noise ratio (SNR), the power of the signal, and others. In a possible further embodiment, the central processing computing system 700 may detect a drift in one or more features from the electrophysiological activity of the neuronal cell culture as a possible signal that the perfusion system no longer operates optimally to sustain the health of the neuronal cell culture.

In a possible embodiment, the central processing computing system 700 may employ a machine learning system trained to extract features from the electrophysiology signals, such as a deep learning tool, a convolution neural network tool (CNN), an artificial neural network tool (ANN), or others. In a possible embodiment, a fully automatic convolutional neural network (CNN) based tool such as AlexNet, VGG, GoogleNet, UNet, Vnet, ResNet or others may be used, but other embodiments are also possible. In a possible embodiment, at regular times ― for instance once every 1 5, 10, 20, 30, 60, or 90 minutes -, the central processing computing system 700 may extract a snapshot from the MEA biochip monitored signals, with the electrophysiology recording module, and analyze the signal to measure a value in accordance with a predefined cell culture stability metrics.

In a possible embodiment, the central processing computing system 700 may employ a multimodal machine learning system to combine the detection of events as may be visible in the cell culture images with the detection of events as may be observable in the electrophysiology signals.

The vision system 760 and/or the MEA monitoring system 770 may raise an alarm for manual intervention depending on the detected features. In a further possible embodiment, the central processing computing system 700 may control the pump system 710 in a closed feedback loop, by receiving measurements and alarms from the computer vision system 760, or from the MEA electrophysiology signal monitoring system 770, or from a flow meter device (not represented), or a combination thereof, and by commanding the pump system with parameters such as the flow direction (push, pull, or alternance of push and pull), the flow rate, the start and end time of each pumping operation in intermittent mode, to react as fast as possible to a triggering event without waiting for manual intervention.

### EXPERIMENTS

FIG. 8 shows four neuronal cell culture monitoring mini-labs operating in parallel, each with its own microfluidics system in accordance with some embodiments of the present disclosure:
- FIG. 8 a) and d): A syringe pump (SyringeOne, NewEra) programmed to push the nutrient liquid at a constant flow of 25µl/hr onto a MEA biochip (MEA1, MEA4) placed upwards and tilted along a first axis so that its outlet, its waste collector repository (Falcon50) and the hose connecting the latter are placed below the MEA biochip device.
- FIG. 8 b): A peristaltic pump (BT100-2J, Longer), programmed to push the nutrient liquid at 0.1rpm (equivalent to 10µl/mn) for 1mn every 10mn onto a MEA biochip (MEA2) placed upwards and tilted along a first axis so that its outlet, its waste collector repository (Falcon50) and the hose connecting the latter are placed below the MEA biochip device.
- FIG. 8 c): A peristaltic pump (BT100-2J, Longer), programmed to push the nutrient liquid at 0.1rpm (equivalent to 10µl/mn) for 1mn every 10mn onto a MEA biochip (MEA3) placed upwards and tilted along a first axis so that its outlet, its waste collector repository (Falcon50) and the hose connecting the latter are placed below the MEA biochip device.

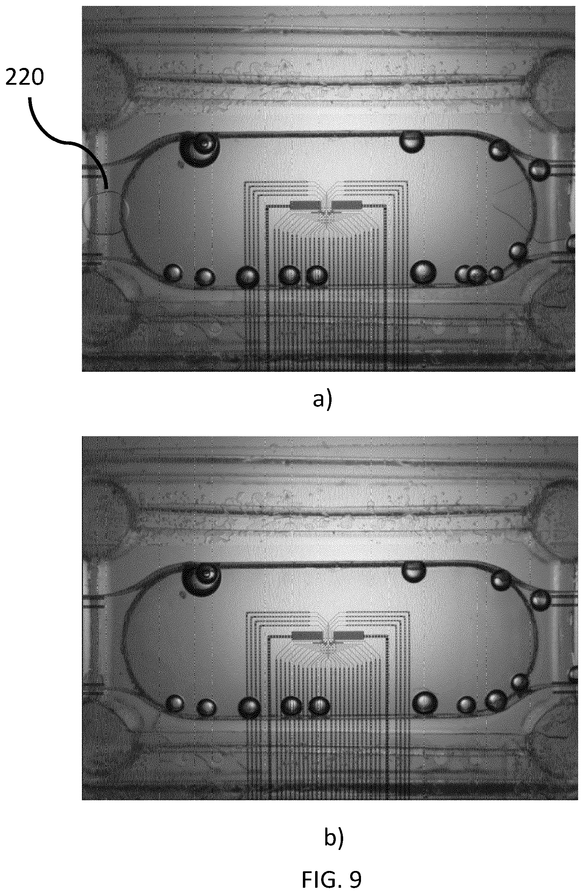

The cell cultures of the MEA biochips of FIG.8 are monitored with a computer vision system which regularly captures images of the cells and the underlying air-liquid interface in order to detect any harmful events such as 2D bubbles, 3D bubbles, or liquid overflows. FIG.9 shows the appearance of a 2D bubble at a first timestamp and its removal at a second timestamp.

In one experiment, a combination of push and pull strategy using the automation system of FIG.7 has also been tested, using the computer vision system 760 to capture images of an MEA cell culture 100 and the central processing computing system 700 to command the pump 710 in a closed-loop mode of automation. The liquid nutrient may be pushed with a peristaltic pump at a rate of 1rpm, corresponding to 35µl /min, for 5mn every 6 hours (corresponding to a total of 175µl/hr, that is half of the volume of the part of the microfluidic channel that lies underneath the air-liquid interface between the edges 260, 270 of the porous membrane 120 as shown in FIG.2). If needed, the peristaltic pump may be programmed to switch to a temporary pull mode at 10-15 rpm for 4-7 sec every hour followed by a push at 1rpm for 5min. These parameters can be adapted according to the metrics (feedback loop). For instance, the central processing computing system 700 receives images captured by the computer vision system and extracts, with a trained artificial neural network tool (ANN), an image of a detected 2D bubble in formation at two different timestamps. or others. The output image of the ANN is post-processed to measure a size metrics of the 2D bubble. If the 2D bubble size falls in the interval between 2 predetermined thresholds, then the algorithm commands to try the temporary pull & push strategy to further remove the 2D bubble.

### OTHER EMBODIMENTS AND APPLICATIONS

As an alternative to the peristaltic pump or the syringe-driver devices, other types of pumps may be used like micro gear pumps, in particular micro annular gear pump, which may simplify maintenance operations for long term 24/7 fully automated operations. Those micro gear pumps have advantages over syringe pump since it avoids any manipulation for refilling the syringe. Micro gear pumps are also more robust to aging compared to peristaltic pumps, since the compression of the tube may lead to crack over time.

As an alternative embodiment to the proposed system arrangement with the pump placed upstream of the MEA biochip to push the liquid towards the air-liquid interface, the pump may be placed on the downstream part of the microfluidics channel to regularly operate in pull mode (aspiration instead of propulsion of the liquid from the liquid dispenser towards the waste collector). With this configuration, the geometry of the system may be arranged to place the microfluidics channel inlet and any upstream components, included but not limited to the liquid dispenser, above the MEA biochip ALI to create a gravity assisted push complement to the pull pumping effort.

While the present disclosure has been primarily described with exemplary embodiments wherein the MEA biochip comprises electrodes 125 directly arranged between an air-liquid interface porous membrane 120 and the cell culture 100, other MEA arrangements are also possible. For instance, the automated cell monitoring system may employ strip-MEAs and/or high density CMOS MEAs arranged separately from the ALI porous membrane of the microfluidics system. In particular, the MEA electrodes do not necessarily lie underneath the cell culture.

While the present disclosure has been primarily described with exemplary embodiments wherein the microfluidics channel underneath the air-liquid interface and the air liquid interface are integrated on a rigid plastic support of the biochip device (FIG. 1), other arrangements are also possible. In a possible alternate embodiment, the air-liquid interface and the microfluidics channel may be made of flexible materials to better adjust to the shape of the cell culture organoids.

While the present disclosure has been primarily described with exemplary embodiments wherein the inlet and the outlet of the microfluidics channel of the biochip device are vertically arranged (top- loading) (FIG. 1), other arrangements are also possible. In a possible alternate embodiment, the inlet and/or the outlet may be arranged parallel to the microfluidics channel (in-line). More generally, the inlet may be any element facilitating the entry of the liquid into the microfluidics channel towards the air-liquid interface, and the outlet may be any element facilitating the release of the liquid out from the microfluidics channel away from the air-liquid interface.

While various embodiments have been described above, it should be understood that they have been presented by way of example and not limitation. It will be apparent to persons skilled in the relevant art(s) that various changes in form and detail can be made therein without departing from the spirit and scope. In fact, after reading the above description, it will be apparent to one skilled in the relevant art(s) how to implement alternative embodiments.

Although the detailed description above contains many specific details, these should not be construed as limiting the scope of the embodiments but as merely providing illustrations of some of several embodiments.

While various embodiments have been described above, it should be understood that they have been presented by way of example and not limitation. It will be apparent to persons skilled in the relevant art(s) that various changes in form and detail can be made therein without departing from the spirit and scope. In fact, after reading the above description, it will be apparent to one skilled in the relevant art(s) how to implement alternative embodiments.

In addition, it should be understood that any figures which highlight the functionality and advantages are presented for example purposes only. The disclosed methods are sufficiently flexible and configurable such that they may be utilized in ways other than that shown.

Although the term "at least one" may often be used in the specification, claims and drawings, the terms "a", "an", "the", "said", etc. also signify "at least one" or "the at least one" in the specification, claims and drawings.

Throughout this specification, plural instances may implement components, operations, or structures described as a single instance. Although individual operations of one or more methods are illustrated and described as separate operations, one or more of the individual operations may be performed concurrently, and nothing requires that the operations be performed in the order illustrated. Structures and functionality presented as separate components in example configurations may be implemented as a combined structure or component. Similarly, structures and functionality presented as a single component may be implemented as separate components. These and other variations, modifications, additions, and improvements fall within the scope of the subject matter herein.

Certain embodiments are described herein as including logic or a number of components, modules, units, or mechanisms. Modules or units may constitute either software modules (e.g., code embodied on a machine-readable medium or in a transmission signal) or hardware modules. A "hardware module" is a tangible unit capable of performing certain operations and may be configured or arranged in a certain physical manner. In various example embodiments, one or more computer systems (e.g., a standalone computer system, a client computer system, or a server computer system) or one or more hardware modules of a computer system (e.g., a processor or a group of processors) may be configured by software (e.g., an application or application portion) as a hardware module that operates to perform certain operations as described herein.

In some embodiments, a hardware module may be implemented mechanically, electronically, biologically or any suitable combination thereof. For example, a hardware module may include dedicated circuitry or logic that is permanently configured to perform certain operations. For example, a hardware module may be a special-purpose processor, such as a field-programmable gate array (FPGA) or an ASIC. A hardware module may also include programmable logic or circuitry that is temporarily configured by software to perform certain operations. For example, a hardware module may include software encompassed within a general-purpose processor or other programmable processor. It will be appreciated that the decision to implement a hardware module mechanically, in dedicated and permanently configured circuitry, or in temporarily configured circuitry (e.g., configured by software) may be driven by cost and time considerations. As synthetic biology develops, all or part of the hardware module may be made of biological cells, such as neurospheres and/or genetically engineered cells (also known as wetware).

The various operations of example methods described herein may be performed, at least partially, by one or more processors that are temporarily configured (e.g., by software) or permanently configured to perform the relevant operations. Whether temporarily or permanently configured, such processors may constitute processor-implemented modules that operate to perform one or more operations or functions described herein. As used herein, "processor-implemented module" refers to a hardware module implemented using one or more processors.

Similarly, the methods described herein may be at least partially processor-implemented, a processor being an example of hardware. For example, at least some of the operations of a method may be performed by one or more processors or processor-implemented modules.

Some portions of the subject matter discussed herein may be presented in terms of algorithms or symbolic representations of operations on data stored as bits or binary digital signals within a machine memory (e.g., a computer memory). Such algorithms or symbolic representations are examples of techniques used by those of ordinary skill in the data processing arts to convey the substance of their work to others skilled in the art. As used herein, an "algorithm" is a self-consistent sequence of operations or similar processing leading to a desired result. In this context, algorithms and operations involve physical manipulation of physical quantities.

Although an overview of the inventive subject matter has been described with reference to specific example embodiments, various modifications and changes may be made to these embodiments without departing from the broader spirit and scope of embodiments of the present invention. For example, various embodiments or features thereof may be mixed and matched or made optional by a person of ordinary skill in the art. Such embodiments of the inventive subject matter may be referred to herein, individually or collectively, by the term "invention" merely for convenience and without intending to voluntarily limit the scope of this application to any single invention or inventive concept if more than one is, in fact, disclosed.

The embodiments illustrated herein are believed to be described in sufficient detail to enable those skilled in the art to practice the teachings disclosed. Other embodiments may be used and derived therefrom, such that structural and logical substitutions and changes may be made without departing from the scope of this disclosure. The Detailed Description, therefore, is not to be taken in a limiting sense, and the scope of various embodiments is defined only by the appended claims, along with the full range of equivalents to which such claims are entitled.

Moreover, plural instances may be provided for resources, operations, or structures described herein as a single instance. Additionally, boundaries between various resources, operations, modules, engines, and data stores are somewhat arbitrary, and particular operations are illustrated in a context of specific illustrative configurations. Other allocations of functionality are envisioned and may fall within a scope of various embodiments of the present invention. In general, structures and functionality presented as separate resources in the example configurations may be implemented as a combined structure or resource. Similarly, structures and functionality presented as a single resource may be implemented as separate resources. These and other variations, modifications, additions, and improvements fall within a scope of embodiments of the present invention as represented by the appended claims. The specification and drawings are, accordingly, to be regarded in an illustrative rather than a restrictive sense.

## Claims

1. A microfluidic system for maintaining of at least one cell culture alive over an air-liquid interface, comprising:
- a microfluidics channel arranged to provide a perfusion of liquid to said cell culture through one or more porous membranes of the air-liquid interface,
- a pumping device arranged to circulate a liquid through the microfluidics channel, **characterized in that**:
- the pumping device is adapted to push a liquid through an inlet of the microfluidics channel, then through an area of the microfluidics channel that is under the air-liquid interface, then through an outlet of the microfluidics channel.

2. The microfluidic system of claim 1, further **characterized in that** the outlet of the microfluidics channel is placed at the same height or lower than the porous membranes of the air-liquid interface in the microfluidics system.

3. The microfluidic system of claim 2, further comprising a waste collector to collect the liquid pushed from the outlet of the microfluidics channel, **characterized in that** the waste collector and any microfluidics elements connecting the outlet to the waste collector are placed at the same height or lower than the porous membranes of the air-liquid interface in the microfluidic system.

4. The microfluidic system of any of the preceding claims, **characterized in that** the microfluidic channel and the air-liquid interface are tilted such that the microfluidics channel outlet is placed at the same height or lower than the porous membranes of the air-liquid interface.

5. The microfluidic system of claim 4, **characterized in that** the microfluidic channel and the air-liquid interface are arranged on a rigid planar support in the microfluidic system, and the rigid planar support is tilted such that the microfluidics channel outlet is placed at the same height or lower than the porous membranes of the air-liquid interface.

6. The microfluidic system of any of the preceding claims, **characterized in that** the inlet of the microfluidic channel is placed lower than the outlet of the microfluidic channel in the microfluidic system.

7. The microfluidic system of claim 6, **characterized in that** the microfluidic channel is tilted such that the inlet of the microfluidic channel is placed lower than the outlet of the microfluidic channel in the microfluidic system.

8. The microfluidic system of claim 7, **characterized in that** the microfluidic channel is arranged on a rigid planar support in the microfluidic system, and the rigid planar support is tilted such that the inlet of the microfluidic channel is placed lower than the outlet of the microfluidic channel in the microfluidic system.

9. The microfluidic system of any of the preceding claims, wherein the pumping device is a programmable peristaltic pump, syringe-driver, or microgear pump.

10. The microfluidic system of claim 9, wherein the pumping device is programmed to push the liquid at a mean flow rate between around 10µl/hr and around 100µl/hr.

11. The microfluidic system of claim 10, wherein the pumping device is programmed to push the liquid at an intermittent flow rate of around 10µl/mn for around 1mn every 10mn.

12. The microfluidic system of any of the preceding claims, further comprising a computer vision image processing system and a central processing computing system, adapted to:
- Detect, with the computer vision image processing system, an image feature;
- Program, with the central processing computing system, the pumping device flow direction, flow rate and flow duration according to the detected image feature.

13. The microfluidic system of claim 12, further adapted to:
- Detect, with a trained machine learning model, an air bubble or a liquid overflow.
- Program, with the central processing computing system, the pumping device flow direction, flow rate and flow duration with a predetermined configuration according to the detected air bubble or liquid overflow.

14. The microfluidic system of claim 13, further adapted to:
- Program, with the central processing computing system, the pumping device flow direction, flow rate and flow duration according to a configuration learnt by the trained machine system in response to the detected air bubble or liquid overflow.

15. The microfluidic system of any of the preceding claims, further comprising an electrophysiology signal processing system, adapted to:
- Detect, with the electrophysiology signal processing system, a signal feature of the cell culture electrophysiological signal;
- Program the pumping device flow direction, flow rate and flow duration according to the detected signal feature.
